# EUROPEAN PATENT APPLICATION

(11) **EP 3 949 849 A1**
(43) Date of publication of application: **09.02.2022**
(21) Application number: 20806592.0
(22) Date of filing: 13.05.2020
(51) Int. Cl.: A61B 5/042

(54) **SMART WEARABLE DEVICE**

(30) Priority: 14.05.2019 CN 201910402154
(71) Applicant: Huawei Technologies Co., Ltd., Shenzhen, Guangdong 518129 (CN)
(72) Inventor: YAO, Yuliang, Shenzhen, Guangdong 518129 (CN); GUO, Zhi, Shenzhen, Guangdong 518129 (CN); WANG, Le, Shenzhen, Guangdong 518129 (CN); LU, Nan, Shenzhen, Guangdong 518129 (CN); YANG, Rongguang, Shenzhen, Guangdong 518129 (CN); XI, Yi, Shenzhen, Guangdong 518129 (CN); SUN, Shiyou, Shenzhen, Guangdong 518129 (CN)
(74) Representative: Gill Jennings & Every LLP
(86) International application number: PCT/CN2020/090019
(87) International publication number: WO 2020/228737

(57) **Abstract**

A smart wearable device is provided, and relates to the technical field of communications devices. The smart wearable device includes a display screen (1) and a rear cover (2) that engages with the display screen (1), and further includes a light generator (3) and a light receiver (5). The light generator (3) and the light receiver (5) are disposed in a mounting cavity formed after the display screen (1) engages with the rear cover (2), the light generator (3) is configured to emit light to an outer side of the rear cover (2), the light receiver (5) is configured to receive light transmitted from the outer side of the rear cover (2), the light generator (3) is disposed on a first control panel, the light receiver (5) is disposed on a second control panel, and the light generator (3) is disposed closer to the rear cover (2) than the light receiver (5).

## Description

This application claims priority to Chinese Patent Application No. 201910402154.6, filed with the China National Intellectual Property Administration on May 14, 2019 and entitled "SMART WEARABLE DEVICE", which is incorporated herein by reference in its entirety.

### TECHNICAL FIELD

This application relates to the technical field of communications devices, and in particular, to a smart wearable device.

### BACKGROUND

As an informatization level rapidly improves and people attach more importance to physical and mental health, requirements for and applications of a smart wearable device that can monitor a physical health status in real time gradually increase. As the most important detection element in the wearable device, a heart rate monitoring element needs to perform monitoring in real time 24 hours a day in increasingly more application scenarios.

Currently, photoplethysmography (Photo Plethysmography, PPG for short), referred to as a photovoltaic method for short, is mainly used in the smart wearable device for heart rate monitoring. Such a heart rate monitoring method is based on a principle of absorption of light by a substance. To be specific, a light-emitting diode (Light-Emitting Diode, LED) and a photodiode (Photo Diode, PD) of the smart wearable device irradiate a blood vessel for a period of time, and a heart rate is measured based on absorbance of blood. Specifically, when a beam on a specific wavelength that is emitted by the LED is irradiated on a skin surface, the beam is transmitted to the PD through transmission or reflection. In this process, due to attenuation of absorption by a skin muscle and the blood, intensity of light detected by the PD decreases. Reflection of skin, bones, flesh, and fat of a human body to light is a fixed value, and volumes of capillaries, arteries, and veins ceaselessly increase or decrease with pulse under the action of the heart. When the heart contracts, a peripheral blood volume is the largest, light absorption is the largest, and the intensity of the light detected by the PD is the lowest. When the heart relaxes, the contrary is the case, and the intensity of the light detected by the PD is the highest. In this way, the intensity of the light received by the PD is in a pulsatile change accordingly.

Currently, a reflective photovoltaic method is a most commonly used method for detecting a heart rate. In the reflective photovoltaic method, a light-emitting diode and a photodiode are located on a same side of a to-be-measured part, and reflected light is mainly measured. Advantages of this method for measuring the heart rate are as follows: The method is quite simple, and there are very low requirements for the to-be-measured part, and nearly all positions with comparatively smooth tissue and little subcutaneous fat can be measured, for example, the forehead and the wrist. Therefore, this method is used in most smart wearable devices such as a smart band and a smartwatch to measure the heart rate.

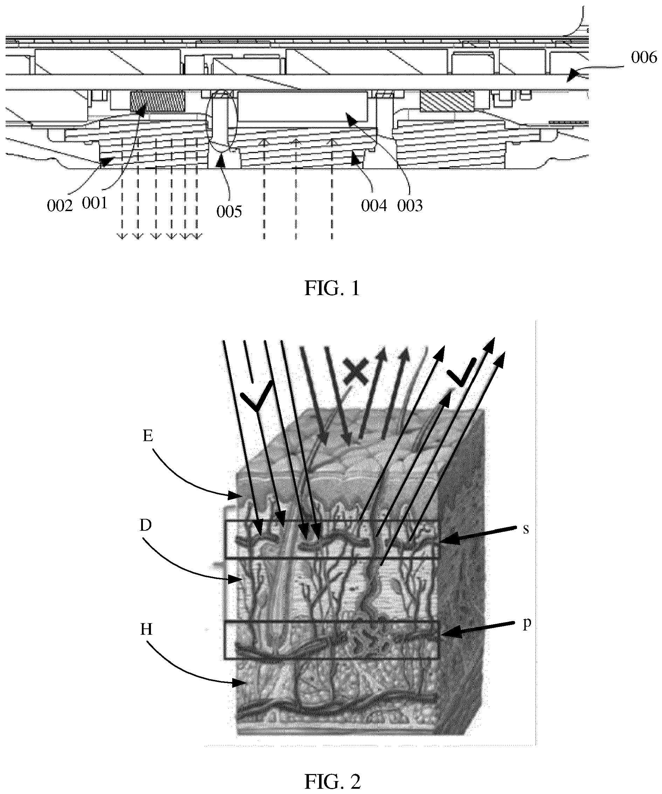

Currently, there is a smart wearable device that can detect a heart rate. The smart wearable device includes a display screen and a rear cover that are disposed opposite to each other. As shown in FIG. 1, the smart wearable device further includes a light-emitting diode 001 disposed in a mounting cavity formed after the display screen engages with the rear cover, and a photodiode 003 that is disposed in parallel with the light-emitting diode 001 and mounted on a same circuit board 006 as the light-emitting diode 001. A shielding structure 005 used for preventing light emitted by the light-emitting diode 001 from being directly irradiated to the photodiode 003 is disposed between the light-emitting diode 001 and the photodiode 003. A corresponding LED lens 002 and a corresponding PD lens 004 are disposed on sides that are of the light-emitting diode 001 and the photodiode 003 and that are opposite to a to-be-measured part. The LED lens 002 is usually inlaid in an opening of the rear cover. Due to a requirement of an assembly process, there is a spacing between the light-emitting diode 001 and the LED lens 002. During specific working, as shown in FIG. 2, the light emitted by the light-emitting diode 001 passes through the LED lens 002 and enters the dermis D and the subcutis H through the epidermis E, and after being reflected by surface capillaries s in the dermis D and deep capillaries p in the subcutis H, the light is received by the photodiode 003 through the PD lens 004.

In the prior art, usually, a thickness of the LED lens 002 is approximately 0.8 mm, and the spacing between the light-emitting diode 001 and the LED lens 002 is approximately 0.3 mm. Consequently, comparatively large power consumption is caused in a process in which the light emitted by the light-emitting diode 001 passes through a comparatively thick LED lens 002, and this affects transmittance of the light emitted by the light-emitting diode 001. However, to ensure a detection effect, irradiation power of the light-emitting diode 001 needs to be increased, and consequently, power consumption of the light-emitting diode 001 is relatively large, and real-time monitoring 24 hours a day cannot be supported. This finally affects product endurance.

### SUMMARY

Embodiments of this application provide a smart wearable device. A main objective is to provide a smart wearable device that can improve transmittance of light emitted by a light generator and reduce power consumption caused when the light emitted by the light generator is transmitted to a to-be-measured part, to further improve endurance of the smart wearable device.

To achieve the foregoing objective, the following technical solutions are used in the embodiments of this application.

According to a first aspect, this application provides a smart wearable device. The smart wearable device includes a display screen and a rear cover that engages with the display screen, and further includes: a light generator and a light receiver, where the light generator and the light receiver are disposed in a mounting cavity formed after the display screen engages with the rear cover, the light generator is configured to emit light to an outer side of the rear cover, the light receiver is configured to receive light transmitted from the outer side of the rear cover, the light generator is disposed on a first control panel, the light receiver is disposed on a second control panel, and the light generator is disposed closer to the rear cover than the light receiver.

In the smart wearable device provided in this embodiment of this application, the light generator is disposed on the first control panel, the light receiver is disposed on the second control panel, and the light generator is disposed closer to the rear cover than the light receiver. In this way, when the light emitted by the light generator passes through the rear cover and is irradiated to a to-be-measured part, a path through which the light passes is shortened in comparison with a path through which the light passes in the prior art, transmittance of the light emitted by the light generator is improved, and illumination loss caused by the light in an illumination process is further reduced. Therefore, endurance of the entire smart wearable device is improved, and use performance of the entire smart wearable device is finally improved.

In a possible implementation of the first aspect, the smart wearable device further includes a package structure configured to package the light generator, a placement cavity is formed inside the package structure, the light generator is disposed in the placement cavity, a surface of the package structure that is opposite to a light-emitting surface of the light generator is a transparent surface, and the transparent surface of the package structure is mounted at an opening provided on the rear cover. The light generator is disposed in the placement cavity of the package structure, and then the package structure is mounted at the opening of the rear cover. In this way, while it is ensured that the light generator is closer to the rear cover and it is therefore ensured that the light generator is closer to the to-be-measured part, the light generator and the package structure are integrated to form a module, and this facilitates mounting.

In a possible implementation of the first aspect, the light-emitting surface of the light generator is attached to the transparent surface. The light-emitting surface of the light generator is attached to the transparent surface, in other words, there is no gap between the light-emitting surface of the light generator and the transparent surface. In this way, a reflection phenomenon caused when the light emitted by the light generator is irradiated onto the transparent surface can be avoided, to further improve transmittance of the light.

In a possible implementation of the first aspect, a material of the package structure is a transparent material, and a light shielding structure is disposed on a surface of the package structure that is opposite to the light receiver. When the light shielding structure is used, the following phenomenon can be effectively prevented: The light emitted by the light generator passes through the package structure and is scattered to the light receiver, and the light receiver directly receives the light emitted by the light generator, an interference signal is formed, and a useful signal is submerged.

In a possible implementation of the first aspect, a side face of the package structure is a step structure. The side face of the package structure is set to the step surface, to add a waterproof path, so that waterproof performance of the entire smart wearable device is improved.

In a possible implementation of the first aspect, the smart wearable device further includes a package structure configured to package the light generator, a material of the package structure is a transparent material, a light-emitting surface of the light generator faces the package structure, the light generator is connected to the package structure by using a transparent adhesive layer, and the package structure is mounted at an opening provided on the rear cover. The package structure is bonded to the package structure by using the transparent adhesive layer, so that while it is ensured that the light generator is closer to the rear cover and it is therefore ensured that the light generator is closer to the to-be-measured part, a connection structure is simple, and implementation is facilitated.

In a possible implementation of the first aspect, the transparent adhesive layer is arranged along an edge of the light-emitting surface of the light generator, and a light shielding structure is disposed on a side wall of the transparent adhesive layer that is opposite to the light receiver. While it is ensured that the transparent adhesive layer connects the package structure and the light generator, the transparent adhesive layer is arranged along the edge of the light-emitting surface of the light generator, and then, the light shielding structure is disposed on the side wall of the transparent adhesive layer that is opposite to the light receiver, to effectively avoid, by using the light shielding structure, a phenomenon that the light emitted by the light generator passes through the transparent adhesive layer and is scattered to the light receiver and the light receiver directly receives the light, an interference signal is formed, and a useful signal is submerged.

In a possible implementation of the first aspect, the light shielding structure is a light shielding film layer. When the light shielding film layer is used as the light shielding structure, a simple structure is implemented, implementation is facilitated, and manufacturing costs are also relatively low.

In a possible implementation of the first aspect, a unidirectional transparent structure is disposed on a side of the package structure that is away from the light-emitting surface of the light generator, and the unidirectional transparent structure is configured to transmit light emitted by the light generator out of the package structure, and is configured to prevent light outside the package structure from passing through the package structure. It is ensured, by using the unidirectional transparent structure, that the light emitted by the light generator can be transmitted, and an internal structure is invisible from the outside. This improves aesthetic appearance of the entire smart wearable device.

In a possible implementation of the first aspect, the unidirectional transparent structure is a unidirectional transparent film layer disposed on the side of the package structure that is away from the light-emitting surface of the light generator. When the unidirectional transparent film layer is used as the unidirectional transparent structure, a structure of the unidirectional transparent structure is simple and easy to implement while it is ensured that the internal structure is invisible from the outside.

In a possible implementation of the first aspect, the side of the package structure that is away from the light-emitting surface of the light generator is a curved surface that is bent in a direction away from the rear cover. The side of the package structure that is away from the light-emitting surface of the light generator is the curved surface that is bent in the direction away from the rear cover, so that it is easier to attach the package structure to the to-be-measured part, and further, the light generator is made closer to the to-be-measured part. This prevents a reflection phenomenon that directly occurs on light irradiated to the to-be-measured part, and avoids a case in which directly emitted light interferes with light absorbed by the to-be-measured part and even submerges a useful signal.

In a possible implementation of the first aspect, a material of the package structure is selected from transparent materials such as transparent ceramic, transparent glass, transparent plastic, and transparent gem.

In a possible implementation of the first aspect, there are a plurality of light receivers, the plurality of light receivers are arranged in a regular shape, and the light generator is disposed at a central position of the plurality of light receivers in the regular shape; or there are a plurality of light generators, the plurality of light generators are arranged in a regular shape, and the light receiver is disposed at a central position of the plurality of light generators in the regular shape.

### BRIEF DESCRIPTION OF DRAWINGS

FIG. 1 is a sectional view of a partial structure of a smart wearable device in the prior art;
FIG. 2 is a diagram of an optical path, at a to-be-measured part, of light emitted by a smart wearable device;
FIG. 3 is a three-dimensional diagram of a smart wearable device according to an embodiment of this application;
FIG. 4 is a schematic diagram of an internal structure of FIG. 3;
FIG. 5 is a three-dimensional diagram of a package structure according to an embodiment of this application;
FIG. 6 is a view from another perspective of FIG. 5;
FIG. 7 is a view from another perspective of FIG. 5;
FIG. 8 is a schematic diagram of an internal structure of FIG. 5; and
FIG. 9 is a schematic diagram of an internal structure of a smart wearable device according to an embodiment of this application.

### DESCRIPTION OF EMBODIMENTS

Embodiments of this application relate to a smart wearable device. Concepts involved in the foregoing embodiments are briefly described below.

### Printed circuit board (Printed Circuit Board, PCB).

### Light-emitting diode (Light-Emitting Diode, LED).

### Photodiode (Photo Diode, PD).

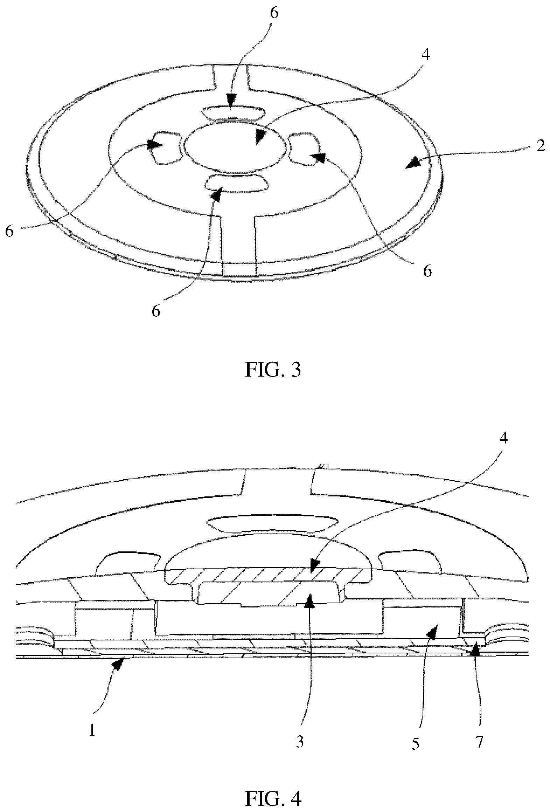

As shown in FIG. 3, FIG. 4, and FIG. 9, an embodiment of this application provides a smart wearable device. The smart wearable device includes a display screen 1 and a rear cover 2 that engages with the display screen 1, and further includes a light generator 3 and a light receiver 5. The light generator 3 and the light receiver 5 are disposed in a mounting cavity formed after the display screen 1 engages with the rear cover 2, the light generator 3 is configured to emit light to an outer side of the rear cover 2, the light receiver 5 is configured to receive light transmitted from the outer side of the rear cover 2, the light generator 3 is disposed on a first control panel (not shown in the figures), the light receiver 5 is disposed on a second control panel 7, and the light generator 3 is disposed closer to the rear cover 2 than the light receiver 5.

The light generator 3 and the light receiver 5 are respectively disposed on the corresponding first control plate and second control plate 7, and then the light generator 3 is disposed closer to the rear cover 2 than the light receiver 5. In this way, a distance between the light generator 3 and the rear cover 2 is reduced in comparison with the prior art, and therefore, a distance between the light generator 3 and a to-be-measured part is reduced. When the light emitted by the light generator 3 is irradiated to the to-be-measured part, a path through which the light passes is shortened in comparison with a path through which the light passes in the prior art, so that illumination loss caused by the light in an illumination process is reduced. Therefore, transmittance of the light emitted by the light generator 3 is transmitted, and endurance of the entire smart wearable device is improved. In addition, the light generator 3 and the light receiver 5 are not located on a same plane, so that mutual light leakage impact between the light generator 3 and the light receiver 5 is greatly weakened, and a phenomenon in which normal reception of a reflected signal by the light receiver 5 is interfered with and affected is also weakened. If the smart wearable device has a heart rate monitoring function, heart rate monitoring accuracy can be significantly improved, and finally, use performance of the entire smart wearable device is improved, and user experience is improved.

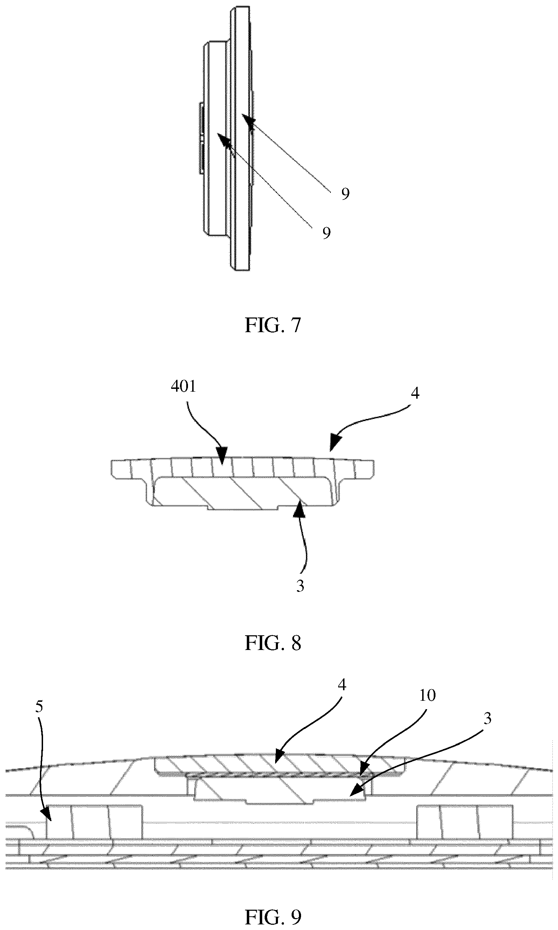

There are a plurality of packaging manners for the light generator 3. In some implementations, as shown in FIG. 3, FIG. 4, and FIG. 8, this application further includes a package structure 4 configured to package the light generator 3. A placement cavity is formed inside the package structure 4, the light generator 3 is disposed in the placement cavity, a surface of the package structure 4 that is opposite to the light-emitting surface of the light generator 3 is a transparent surface 401, and the transparent surface 401 of the package structure 4 is mounted at an opening provided on the rear cover 2. In some other implementations, as shown in FIG. 9, this application further includes a package structure 4 configured to package the light generator 3. A material of the package structure is a transparent material, the light-emitting surface of the light generator 3 faces the package structure 4, the light generator 3 is connected to the package structure 4 by using a transparent adhesive layer 10, and the package structure 4 is mounted at an opening provided on the rear cover 2. Certainly, this application further includes another package structure of the light generator 3.

When the package structure shown in FIG. 3, FIG. 4, and FIG. 8 is used, the light emitted by the light generator 3 passes through the transparent surface 401 of the package structure 4, and further passes through the rear cover 2 and is irradiated to the to-be-measured part.

In the package structure shown in FIG. 3, FIG. 4, and FIG. 8, the placement cavity is formed inside the package structure 4, and the light generator 3 is directly disposed in the placement cavity, in other words, the package structure 4 is used to package the light generator 3 by wrapping the light generator 3. Therefore, in specific implementation, the light generator 3 may be first mounted in the package structure 4, and then the light generator 3 and the package structure 4 become an independent module, and then the module is mounted at the opening provided on the rear cover 2. In this packaging manner, while it is ensured that an optical path on which the light emitted by the light generator 3 is irradiated to the to-be-measured part is shortened, the package structure 4 and the light generator 3 may be integrated. Compared with the prior art, an assembly avoidance gap between the light generator 3 and a lens of the light generator is omitted.

When the smart wearable device is used as a heart rate detection device, it is required to ensure as far as possible that a light reflection phenomenon does not occur on the light (as shown in FIG. 2, a mark × in FIG. 2 indicates the light reflection phenomenon) in a process in which the light emitted by the light generator 3 is irradiated to the to-be-measured part. This is because when the light is reflected, not only transmittance is reduced, but the reflected light also becomes an interference signal, and consequently reception of a useful signal by the light receiver 5 is affected. Therefore, to improve heart rate measurement accuracy of the smart wearable device, it is ensured as far as possible that the refection phenomenon does not occur on the light in the process in which the light emitted by the light generator 3 is irradiated to the to-be-measured part. Generally, in the process in which the light emitted by the light generator 3 is irradiated to the to-be-measured part, there are two light reflection cases. One is that a gap between the light generator 3 and the package structure 4 causes light reflection, and the other is that a gap between the package structure 4 and the to-be-measured part causes light reflection.

To prevent light reflection caused by the gap between the light generator 3 and the package structure 4, as shown in FIG. 8, the light-emitting surface of the light generator 3 is attached to the transparent surface 401. In this way, when light emitted from the light-emitting surface of the light generator 3 directly passes through the transparent surface 401, a reflection phenomenon does not occur on the transparent surface 401. In this way, not only transmittance of the light is improved, but accuracy of measured data is ensured.

Similarly, to prevent light reflection caused by the gap between the package structure 4 and the to-be-measured part, as shown in FIG. 8 (in FIG. 2, a mark √ indicates that no reflection phenomenon occurs on the light), a side, exposed on the rear cover 2, of the package structure 4 that is away from the light-emitting surface of the light generator 3 is a curved surface that is bent in a direction away from the rear cover 2. By using the curved surface, the package structure 4 may be tightly attached to the to-be-measured part, and a reflection phenomenon does not occur on the to-be-measured part. In this way, not only transmittance of the light is improved, but reflected light is also prevented from interfering with light that enters the light receiver.

In the foregoing embodiment, the surface of the package structure 4 that is opposite to the light-emitting surface of the light generator 3 is the transparent surface, to ensure that the light emitted by the light generator 3 is irradiated to the to-be-measured part. However, in specific implementation, an entirely transparent package structure 4 is usually directly used. In this way, it is convenient to draw materials, and costs are also reduced correspondingly. For example, the material of the package structure 4 may be selected from a transparent material, and the package structure 4 may be selected from, including but not limited to, transparent ceramic, transparent glass, transparent plastic, and transparent sapphire.

When the package structure shown in FIG. 3, FIG. 4, and FIG. 8 is used, the light generator 3 and the light receiver 5 are not located on a same plane, and mutual light leakage impact between the light generator 3 and the light receiver 5 is greatly weakened, but to further prevent the light emitted by the light generator 3 from being leaked to the light receiver 5 and affecting light reception of the light receiver 5, a light shielding structure is disposed on a surface of the entirely transparent package structure 4 that is opposite to the light receiver 5. When the light shielding structure is disposed, the light emitted by the light receiver 5 is prevented from passing through the package structure 4 and being scattered to the light receiver 5. In addition, a light shielding structure may be disposed on a surface of the light receiver 5 that is opposite to the light generator 3, or a light shielding structure may be disposed between the light generator 3 and the light receiver 5. In conclusion, any light shielding structure that can prevent light leakage between the light generator 3 and the light receiver 5 falls within the protection scope of this application.

There are a plurality of implementations for the light shielding structure. For structure simplicity and convenience of implementation, as shown in FIG. 7, the light shielding structure is a light shielding film layer 9. In specific implementation, the light shielding film layer 9 needs to be coated only on a side of the package structure 4 that is close to the light receiver 5. However, to simplify a coating process, the light shielding film layer 9 may be coated on an entire side face of the package structure 4. For example, the light shielding film layer 9 may be a black film layer or may be another dark-color film layer. Certainly, a light shielding structure of another structure may be used. However, the light shielding film layer 9 is a better solution than another light shielding structure.

Generally, the smart wearable device is a smartwatch, a smart band, or the like, and is prone to be in contact with water in use. Therefore, waterproof performance of the smart wearable device is also a key factor for performance of the smart wearable device. In this application, to enable the smart wearable device to have a waterproof function, a waterproof function is improved by adding a water inlet path. As shown in FIG. 8, a side face of the package structure 4 is a step structure. When the package structure 4 is mounted and inlaid on the rear cover 2, the side face of the package structure is set to the step surface, to add a water inlet path from the outer side of the rear cover 2 to an inner side of the rear cover, and in addition, the light generator 3 is disposed in the placement cavity of the package structure 4, and the light generator 3 also plays a protective role. Therefore, waterproof performance of the entire smart wearable device is effectively improved by using both the step structure and the placement cavity.

In this embodiment of this application, the transparent surface of the package structure 4 is directly inlaid on the rear cover 2, in other words, is directly exposed. When an entirely transparent material is used for the package structure 4, a unidirectional transparent structure is disposed on a side of the package structure 4 that is away from the light-emitting surface of the light generator 3, and the unidirectional transparent structure is configured to transmit the light emitted by the light generator 3 out of the package structure 4, and is configured to prevent light outside the package structure 4 from passing through the package structure 4. In this way, it can be ensured that the light generator 3 passes through the package structure 4 and is irradiated only to the to-be-measured part, but the light generator 3 inside the smart wearable device is invisible from the outside. This improves aesthetic appearance of the smart wearable device.

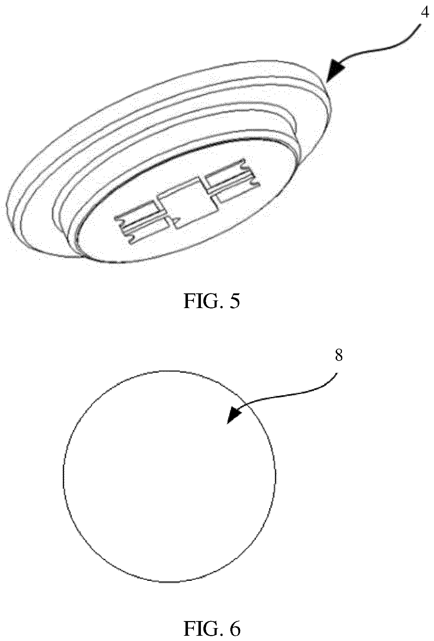

There are a plurality of structures for the unidirectional transparent structure. For structure simplicity and convenience of implementation, as shown in FIG. 6, the unidirectional transparent structure is a unidirectional transparent film layer 8 disposed on the side of the package structure 4 that is away from the light-emitting surface of the light generator 3. The unidirectional transparent film layer 8 is used as the unidirectional transparent structure. In a specific operation, a unidirectional transparent film needs to be coated only on the side of the package structure 4 that is away from the light-emitting surface of the light generator 3, to form the unidirectional transparent film layer 8. Certainly, a unidirectional transparent structure of another structure may be used. However, the unidirectional transparent film layer 8 is a better solution than another unidirectional transparent structure.

The package structure 4 is in a plurality of shapes. In some implementations, as shown in FIG. 5 and FIG. 6, the package structure 4 is a solid of revolution structure. In some other implementations, the package structure 4 is a cuboid structure, and certainly, may be another structure. A specific shape of the package structure is not limited in this application, and any shape falls within the protection scope of this application.

To prevent a case in which relatively large power consumption is caused when the light emitted by the light generator 3 passes through the package structure 4, while compressive strength is ensured, a smaller thickness of the package structure 4 is preferred. For example, when the material of the package structure 4 is any one of transparent ceramic, transparent glass, or transparent plastic, a thickness of a packaging surface of the package structure 4 is 0.3 mm to 0.4 mm; and when the material of the package structure 4 is transparent gem, the thickness of the packaging surface of the package structure 4 is less than 0.3 mm. In comparison with an existing package structure of 0.8 mm, the thickness is obviously reduced, in other words, power consumption is obviously reduced.

When the package structure 4 shown in FIG. 9 is used, in this solution, the package structure 4 is a plate-shaped structure, and the light generator 3 is connected to the package structure 4 by using the transparent adhesive layer 10, so that a simple structure is implemented. In specific implementation, a transparent adhesive may be coated on a side of the package structure 4 that faces the light generator 3 to form the transparent adhesive layer 10, and then the light generator 3 is bonded to the transparent adhesive, to prevent a light reflection phenomenon caused by a gap between the light-emitting surface of the light generator 3 and the package structure 4. During specific bonding, the light generator 3 may be made close to the package structure 4 as far as possible, to avoid, as far as possible, a phenomenon that there is a gap between the light generator 3 and the package structure 4. In this way, transmittance of the light is improved, and accuracy of measured data is ensured.

The light generator 3 is bonded to the package structure 4 by using the transparent adhesive layer 10. Therefore, for convenience of material selection, an entirely transparent package structure 4 is directly selected. For example, the material of the package structure 4 may be selected from a transparent material, and the package structure 4 may be selected from, including but not limited to, transparent ceramic, transparent glass, transparent plastic, and transparent sapphire.

In some implementations, to ensure that the smart wearable device has a waterproof function, a side face of the package structure 4 is also designed as a step structure. The side face of the package structure is set to the step surface, to add a water inlet path from the outer side of the rear cover 2 to an inner side of the rear cover.

In the embodiment shown in FIG. 9, the package structure 4 forms a partial structure of the rear cover 2, and is also directly exposed. Similarly, to improve aesthetic appearance of the entire smart wearable device, a unidirectional transparent structure is disposed on a side of the package structure 4 that is away from the light-emitting surface of the light generator 3. The unidirectional transparent structure is configured to transmit the light emitted by the light generator 3 out of the package structure 4, and is configured to prevent light outside the package structure 4 from passing through the package structure 4. For example, the unidirectional transparent structure is a unidirectional transparent film layer 8 disposed on the side of the package structure 4 that is away from the light-emitting surface of the light generator 3. In the embodiment shown in FIG. 9, to prevent light reflection caused by a gap between the package structure 4 and the to-be-measured part, a side, exposed on the rear cover 2, of the package structure 4 that is away from the light-emitting surface of the light generator 3 is a curved surface that is bent in a direction away from the rear cover 2. In this curved surface design, the package structure 4 may be tightly attached to the to-be-measured part, and a reflection phenomenon does not occur on the to-be-measured part. In this way, not only transmittance of the light is improved, but reflected light is also prevented from interfering with light that enters the light receiver.

When the light generator 3 and the package structure 4 are connected by using the transparent adhesive layer 10, the light generator 3 is disposed near the rear cover 2, the light generator 3 and the light receiver 5 are not located on a same plane, and mutual light leakage impact between the light generator 3 and the light receiver 5 is greatly weakened, but to further prevent the light emitted by the light generator 3 from being leaked to the light receiver 5 and affecting light reception of the light receiver 5, the transparent adhesive layer 10 is arranged along an edge of the light-emitting surface of the light generator 3, and a light shielding structure is disposed on a side wall of the transparent adhesive layer 10 that is opposite to the light receiver 5. In this way, it is ensured that the light generator 3 and the package structure 4 are connected by using the transparent adhesive layer 10, and through disposing of the light shielding structure, the light emitted by the light generator 3 is prevented from passing through the transparent adhesive layer 10 and being irradiated to the light receiver 5. In specific implementation, light shielding ink may be coated on an inner wall of a hole provided on the rear cover 2, and the light shielding ink forms the light shielding structure.

For example, the light shielding structure is a light shielding film layer disposed on a side wall of the transparent adhesive layer 10 that is opposite to the light receiver 5. The light shielding film layer provided in this embodiment of this application is used as the light shielding structure. In comparison with an existing retaining wall or light shielding foam that is used as the light shielding structure, a simple structure is implemented, small space is occupied, and a manufacturing process is also simple and convenient, and therefore, manufacturing costs of the entire smart wearable device are reduced.

In some implementations, as shown in FIG. 3, the smart wearable device further includes a light receiver package structure 6 configured to package the light receiver 5. Because the light receiver 5 is in a relatively large size, a manufacturing process is comparatively complex when a manner in which the light receiver 5 is inlaid in the light receiver package structure is used. Therefore, a hole is preferably provided on the rear cover 2, and the light receiver package structure 6 is mounted in the hole. Alternatively, the rear cover 2 is an entirely transparent structure. A light shielding film is first coated on the rear cover 2, and then, hollowing processing is performed at a position on the rear cover 2 that is opposite to a light receiving surface of the light receiver 5 to form the light receiver package structure 6.

To improve and ensure monitoring efficiency of the smart wearable device, as shown in FIG. 3, there are a plurality of light receivers 5, the plurality of light receivers 5 are arranged in a regular shape, and the light generator 3 is disposed at a central position of the plurality of light receivers in the regular shape. In addition, there are a plurality of light generators 3, the plurality of light generators 3 are arranged in a regular shape, and the light receiver 3 is disposed at a central position of the plurality of light generators 5 in the regular shape. Certainly, another manner for arranging the light generator 3 and the light receiver 5 also falls within the protection scope of this application.

The light generator 3 provided in the embodiments of this application may be an LED lamp, the light receiver 5 may be a PD, and the first control panel and the second control panel may be PCB boards.

The smart wearable device provided in the embodiments of this application may be a smartwatch, a smart band, or another wearable device. A specific structure is not limited herein.

In the descriptions of this specification, the described specific features, structures, materials, or characteristics may be combined in a proper manner in any one or more of the embodiments or examples.

The foregoing descriptions are merely specific implementations of the present invention, but are not intended to limit the protection scope of the present invention. Any variation or replacement readily figured out by a person skilled in the art within the technical scope disclo sed in the present invention shall fall within the protection scope of the present invention. Therefore, the protection scope of the present invention shall be subject to the protection scope of the claims.

## Claims

1. A smart wearable device, comprising a display screen and a rear cover that engages with the display screen, and further comprising:
a light generator and a light receiver, wherein the light generator and the light receiver are disposed in a mounting cavity formed after the display screen engages with the rear cover, the light generator is configured to emit light to an outer side of the rear cover, the light receiver is configured to receive light transmitted from the outer side of the rear cover, the light generator is disposed on a first control panel, the light receiver is disposed on a second control panel, and the light generator is disposed closer to the rear cover than the light receiver.

2. The smart wearable device according to claim 1, further comprising a package structure configured to package the light generator, wherein a placement cavity is formed inside the package structure, the light generator is disposed in the placement cavity, a surface of the package structure that is opposite to a light-emitting surface of the light generator is a transparent surface, and the transparent surface of the package structure is mounted at an opening provided on the rear cover.

3. The smart wearable device according to claim 2, wherein the light-emitting surface of the light generator is attached to the transparent surface.

4. The smart wearable device according to claim 2 or 3, wherein a material of the package structure is a transparent material, and a light shielding structure is disposed on a surface of the package structure that is opposite to the light receiver.

5. The smart wearable device according to any one of claims 2 to 4, wherein a side surface of the package structure is a step structure.

6. The smart wearable device according to claim 1, further comprising a package structure configured to package the light generator, wherein a material of the package structure is a transparent material, a light-emitting surface of the light generator faces the package structure, the light generator is connected to the package structure by using a transparent adhesive layer, and the package structure is mounted at an opening provided on the rear cover.

7. The smart wearable device according to claim 6, wherein the transparent adhesive layer is arranged along an edge of the light-emitting surface of the light generator, and a light shielding structure is disposed on a side wall of the transparent adhesive layer that is opposite to the light receiver.

8. The smart wearable device according to claim 4 or 7, wherein the light shielding structure is a light shielding film layer.

9. The smart wearable device according to any one of claims 2 to 8, wherein a unidirectional transparent structure is disposed on a side of the package structure that is away from the light-emitting surface of the light generator, and the unidirectional transparent structure is configured to transmit light emitted by the light generator out of the package structure, and is configured to prevent light outside the package structure from passing through the package structure.

10. The smart wearable device according to claim 9, wherein the unidirectional light transparent structure is a unidirectional transparent film layer disposed on the side of the package structure that is away from the light-emitting surface of the light generator.

11. The smart wearable device according to any one of claims 2 to 10, wherein the side of the package structure that is away from the light-emitting surface of the light generator is a curved surface that is bent in a direction away from the rear cover.
